# EUROPEAN PATENT SPECIFICATION

(11) **EP 1 715 841 B1**
(45) Date of publication and mention of the grant of the patent: **16.05.2007**
(21) Application number: 05707094.8
(22) Date of filing: 26.01.2005
(51) Int. Cl.: A61K 8/81, A61K 8/97, A61Q 13/00

(54) **COSMETIC COMPOSITION COMPRISING WASHOUT RESISTANT FRAGRANCE**
KOSMETISCHE ZUSAMMENSETZUNG MIT AUSWASCH-RESISTENTEM DUFT
COMPOSITION COSMETIQUE DOTEE D'UNE FRAGRANCE ETANCHE A L'EAU

(30) Priority: 27.01.2004 DE 102004005095; 09.08.2004 US 600018 P
(43) Date of publication of application: 02.11.2006
(73) Proprietor: Coty B.V., 2031 CC Haarlem (NL)
(72) Inventor: KULKARNI, Rupali, A., Bridgewater, NJ 08807 (US); SMITH, Leslie, C., Princeton, NJ 08540 (US); KOPF, William, Mine Hill, NJ 07803 (US); GILLIAM, Lisa, D., Scotch Plains, NJ 07076-1613 (US); MACCHIO, Ralph, Sparta, NJ 07971 (US)
(74) Representative: Walter, Wolf-Jürgen
(86) International application number: PCT/EP2005/000931
(87) International publication number: WO 2005/070371

(56) References cited:
- US-A- 6 027 739
- US-B1- 6 172 037
- US-B1- 6 190 681

## Description

The present invention refers to a cosmetic composition which, in particular, enables the perfume component to resist wash off by water and sweat and to remain fixed on the skin for a long time (fragrance-watertight composition).

It is known to encapsulate perfume oils in liposomes or cyclodextrins to achieve a controlled release of these oils. Several systems have also been used to improve bonding to the skin or to extend the time the perfume lasts, e.g. chitin derivatives, quaternary chitosans, silicate materials and thermoplastic polyamides.

US 6143353 A discloses the coating of pharmaceutically active ingredients as well as fragrant components with an aqueous dispersion of a plasticized, hydrophobic acrylic polymer, which acrylic polymer is an (alkyl) ester of acrylic/methacrylic acid.

US 6172037 B1 discloses perfume-fixing substances comprising polyvinylpyrrolidone, hydroxypropyl cellulose and a hydrophobic oil.

US 6 027 739 A discloses smear resistant compositions comprising an alcohol-soluble, water-insoluble polymer, such as carboxylate acrylate/octylacrylamide copolymer. The polymer is used to impart smear resistance, not as fragrance fixer.

The object of the present invention is to provide a cosmetic composition which is wash-off resistant by water and at the same time ensures that the perfume component remains fixed on the skin for a long time.

A further object of the invention is to provide a cosmetic composition with reduced fragrance content and a feeling of the fragrance by the consumer without any difference.

According to the invention, the cosmetic composition comprising fragrances is further comprising a fragrance-fixing complex which comprises
0.01 to 10% by weight of a hydrophobic, alcohol-soluble, carboxylated acrylates/octylacrylamide copolymer,
0.01 to 10% by weight of a hydrolyzed jojoba ester,
and where all percents are related to the total weight of the cosmetic composition.

The range of fragrances is between 0.01 and 35 %by weight. A preferred range is 0.1-16 %, more preferred 0.5-8 %by weight, most preferred 1 to 5 %by weight.
It is preferred that the copolymer be contained in an amount ranging between 0.1 and 5 % by weight, more preferred 0.1-1.0 %by weight. The carboxylated acrylates/octylacrylamide copolymer can be replaced by the acrylic polymer Dermacryl^{®} AQF (National Starch & Chemical Co., Bridgewater NJ, USA).

Further it is preferred that the jojoba ester is present in a range of 0.1 to 5 %, more preferred 0.1-1 %by weight.

In a special embodiement of the invention the jojoba ester and the copolymer constitute a separately prepared complex in the relation 1:1-4, preferably 1:1.2-2.8 of jojoba ester : polymer.

In a further embodiement of the invention the cosmetic composition comprises an alcohol or alcohol/water mixture.

The aforesaid fragrance-fixing complex can be used in cosmetic products such as emulsions, creams, lotions, sprays, shower gels, shower oils, bath products, foam baths, perfumes, aftershaves, shaving balms, face lotions, hair conditioners, skin gels, deodorants, detergents etc. At the same time, further cosmetic auxiliaries and carrier substances as they are commonly used in such preparations can be used, as well as further active agents.Auxiliaries include e.g. preservatives, colourants, polyols, esters, electrolytes, gel-forming agents, polar and nonpolar oils, polymers, further copolymers, stabilizers, surface-active agents.

Cosmetic active agents include e.g. organic sunscreens, scavengers, moisturizing substances, vitamins, enzymes, plant-based active agents, polymers, antioxidants, anti-inflammatory natural active agents.

Preferred cosmetic fragrances are oil-soluble fragrances such as all fragrance oils which are known for the skilled in the art.

The hydrophobic, alcohol-soluble, carboxylated acrylates/octylacrylamide copolymer used according to the invention is soluble in ethanol, isopropanol and fatty alcohols. It can be made water-soluble or dispersible in water by neutralizing the carboxy groups with water-soluble bases such as triethanolamine, sodium carbonate, aminomethylpropanol, potassium hydroxide or ammonium hydroxide. It can thus be easily washed off the skin with soap, if necessary. Due to the interaction of the copolymer with the hydrolyzed jojoba ester, which ester is soluble in the aqueous-alcoholic system and in glycol, and the solvent, fragrant molecules are integrated into a complex and remain bonded to the skin for a very long time. At the same time, the aforesaid complex has a strong water-repellent effect. Thus a very good endurance of the fragrant molecules on the skin is achieved during rain, sweat, swimming, etc.

A preferred copolymer is Acrylates Octylacrylamide Copolymer, with an acidity of 2,4 meq/g. Such a product is available under DERMACRYL®AQF or DERMACRYL®79 by National Starch and Chem. Comp., NJ, USA.

A preferred hydrolyzed jojoba ester is one of plant origin (Simmondsia chinensis) having a saponification value according to AOCS Cd 3-25* of max. 1mg KOH/g, a iodine value according to AOCS Cd. 1d-92* of between 14 and 17g/100g and a trans isomer value according to AOCS Cd 14-95* of max. 0.1% by weight (* test procedure according to American Oil Chemists Society). Such a product (INCI: Hydrolyzed Jojoba Esters (and) Water) is available under Floraesters® K-20W Jojoba by Int. Floratech Technol. Ltd., Hartsdale, NY, USA.

The following substances are particularly suitable for further processing the cosmetic complex.
Oils: Silicone oils, mineral oils, Hydrogenated Polyisobutene, Polyisoprene, Squalane, Tridecyl Trimellitate, Trimethylpropane Triisostearate, Isodecyl Citrate, Neopentyl Glycol Diheptanoate, PPG-15 Stearyl Ether as well as vegetable oils such as Calendula Oil, Jojoba Oil, Avocado Oil, Macadamia Nut Oil, Castor Oil, Cocoa Butter, Coconut Oil, Maize Oil, Cottonseed Oil, Olive Oil, Palm Kernel Oil; Rapeseed Oil, Safflower Oil, Sesame Seed Oil, Soybean Oil, Sunflower Seed Oil, Wheatgerm Oil, Grapeseed Oil, Kukui Nut Oil, Thistle Oil, and mixtures thereof. Synthetic squalane or squalane made from natural products is suitable too, as well as cosmetic esters or ethers which can be branched or linear, saturated or unsaturated.

Scavengers: Antioxidants, vitamins such as Vitamin C and derivatives thereof, e.g. ascorbyl acetate, ascorbyl phosphate and ascorbyl palmitate; Vitamin A and derivatives thereof; folic acid and derivatives thereof; Vitamin E and derivatives thereof such as tocopheryl acetate; flavones and flavonoids; amino acids such as histidine, glycine, tyrosine, tryptophan and derivatives thereof; carotenoids and carotenes such as α-carotene, β-carotene; uric acid and derivatives thereof; α-hydroxy acids such as citric acid, lactic acid, malic acid.
Moisturizing substances: Glycerine, Butylene Glycol, Propylene Glycol, and mixtures thereof:
Organic sunscreens: Derivatives of 4-aminobenzoic acid such as 4-(dimethylamino)-benzoic acid-(2-ethylhexyl) ester; esters of cinnamic acid such as 4-methoxy cinnamic acid(2-ethylhexyl) Ester; benzophenone derivatives such as 2-Hydroxy-4-methoxy benzophenone; derivatives of 3-benzylidene camphor such as 3-Benzylidene Camphor. Other preferred oil-soluble UV filters are Benzophenone-3, Butyl Methoxybenzoylmethane, Octyl Methoxycinnamate, Octyl Salicylate, 4-Methylbenzylidene Camphor, Homosalate, and Octyl Dimethyl PABA.
Surface-active agents: Anionic, amphoteric, non-ionic or cationic surface-active agents, or mixtures thereof. Cationic polymers or a mixture of anionic and amphoteric surface-active agents are particularly preferred.
Non-limiting examples of anionic foaming surface-active agents include those selected from the group consisting of alkyl sulphates, alkyl ether sulphates, sulphated monoglycerides, sulphonated olefins, alkyl aryl sulphonates, primary or secondary alkane sulphonates, alkyl sulphosuccinates, acyl taurates, acyl isothionates, alkyl glyceryl ether sulphonates, sulphonate methyl esters, sulphonated fatty acids, alkyl phosphates, acyl glutamates, acyl sarcosinates, alkyl sulphoacetates, acylated peptides, alkyl ether carboxylates, acyl lactylates of anionic surface-active agents containing fluorine, and mixtures thereof. Mixtures of anionic surface-active agents can be effectively used in the present invention.

Examples of amphoteric surface-active agents which can be used in the present invention include at least those having an acid group. The aforesaid group can be a carboxyl group or a sulphonic acid group. Quaternary nitrogen and therefore quaternary amino acids are included. They should, in general, contain an alkyl group or alkenyl group having 7 to 18 carbon atoms. Suitable amphoteric detergents include simple betaines and amidobetaines which are a mixture of C12- and C14-alkyl groups derived from the coconut so that at least half, preferably three quarters, of the R1-hydrocarbon chain has 10 to 14 carbon atoms. It is preferred that the other two R2- and R3-hydrocarbon chains be methyl. Further, the amphoteric detergent can be a sulphobetaine. Amphoacetates and diamphoacetates can also occur as possible zwitterionic and/or amphoteric compounds, which can be used. An amphoteric surface-active agent should, in general, be contained in an amount ranging approximately between 0.1 and 20%, preferably 5 and 18% by weight, relative to the composition.

Suitable non-ionic surface-active agents include, but are not limited to, Coconut Acyl Monoethanolamide or Coconut Acyl Diethanolamide, Alkyl Polysaccharide, Lactobionamide, Ethylene Glycol Ester, Glycerine Monoether, Polyhydroxyamide (Glucamide), primary and secondary alcohol ethoxylates, particularly C₈₋₂₀ aliphatic alcohols ethoxylated with an average of 1 to 20 moles ethylene oxide per mole of alcohol. Mixtures of the aforesaid surface-active agents can also be used.

Pigments, pigment mixtures or powders with a pigment-like effect, also including those with a pearl-gloss effect may be added to the composition of the invention. The may include, for example, iron oxides, aluminum silicates such as ochre, titanium (di)oxide, mica, kaolin, manganese containing clays such as umber and red bole, calcium carbonate, French chalk, mica-titanium oxide, mica-titanium oxide-iron oxide, bismuth oxychloride, nylon beads, ceramic beads, expanded and non-expanded synthetic polymer powders, powdery natural organic compounds such as milled solid algae, milled plant parts, encapsulated and non-encapsulated cereal starches and mica-titanium oxide-organic dye.

Also included in the composition of the invention are antiperspirants and deodorants, such as Triclosan, Trimethyl Citrate, Farnesol, Aluminum Chlorhydrate, Aluminum Zirconium Tetrachlorhydex GLY etc.

In comparative tests, it has been found that the addition of the complex according to the invention brings about a significant increase in waterproofness during a period of 4-6 hours, both in a sensory test with test persons and in an analytical test by means of gas chromatography/mass spectroscopy. The waterproofness of an aftershave in a test lasting 8 hours was also considerably higher than that of the untreated original product.

It has further been found that the addition of the complex in the range of 0.5-8 % by weight, preferably 1-3 % by weight, related to the total weight of the cosmetic composition, e.g. a perfume, to the other ingredients of the cosmetic composition, can reduce the amount of the fragrance usually added to the composition without loss of fragrance intensity. This means that in consumer studies carried out to determine whether there was a difference in fragrance character between a standard product and a product with 20 % less fragrance and added 1-2 % of the complex of the invention, no significant difference has been stated.

The preferred method for manufacturing the waterproof cosmetic composition consists in that a hydrolyzed jojoba ester is mixed with some alcohol or alcohol/water mixture, and at a temperature ranging between 18-40°C an acrylates/octylacrylamide copolymer in powder form is sprayed into the mixture during the mixing process. The received complex can dried' before use in the cosmetic formulation or can directly added to the other ingredients of of the composition. Mixing of some alcohol, the ester, the copolymer and optionally a 10 % Na₂CO₃ solution in that order is also possible.

The use of the fragrance-fixing complex of the invention is preferred in the following groups of products
- perfumes
- hair care and body care products e.g. hair washes, body washes, shower gels, shampoos, conditioners, etc.
- sun care products e.g. sun creams, sun lotions, after-sun products, body bronzers, sun sprays, sun milks etc.
- antperspirants and deodorants
- products of color cosmetic e.g. mascaras, foundations, make-up, lipsticks, lip balms etc.

The invention will hereinafter be explained in more detail by means of examples. The figures show
Fig. 1: Sensory analysis of aftershave of example 1
Fig. 2: Analytical results of aftershave of example 1

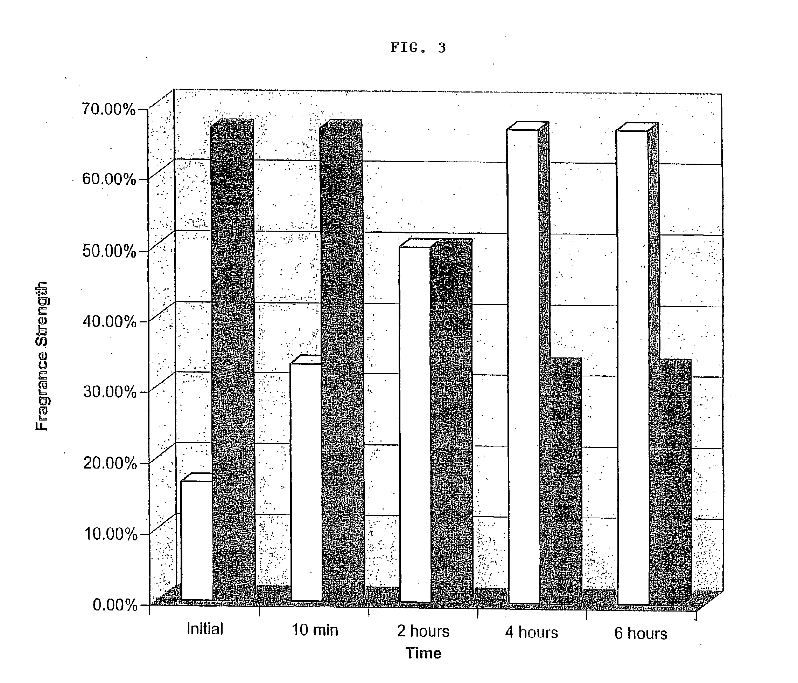

Fig. 3: Sensory analysis of EDT of example 11
Fig. 4: SPME results for fragrance C (ex.16) skintight headspace

All quantities in the examples are given in percent by weight unless indicated otherwise.

### Example 1 Aftershave

| **Phase A** | |
|---|---|
| Ethanol | q.s. ad 100 |
| Octyl Methoxycinnamate | 0.2 |
| Benzophenone-3 | 0.1 |
| PEG-60 Hydrogenated Castor Oil | 1.5 |
| L-Menthol | 0.15 |

| **Phase B** | |
|---|---|
| Fragrance | 7.0 |
| Ethanol | 15.0 |

| **Phase C** | |
|---|---|
| Water | 12 |
| Allantoin | 0.1 |

| **Phase D** | |
|---|---|
| Ethanol | 6.0 |
| Hydrolyzed Jojoba Esters & Water | 0.5 |
| Acrylates/Octylacrylamide Copolymer | 1.0 |

| **Phase E** | |
|---|---|
| Colourants | 0.015 |

The ingredients of Phase A were mixed with one another, incorporating PEG-60 Hydrogenated Castor Oil at approx. 60°C. Separately, Phases B and C were prepared in the same manner. Phase D was prepared by mixing the jojoba ester with ethanol at approx. 25 °C while spraying in the copolymer in powder form during the mixing process. The mixture was then stirred until a clear, homogeneous solution was obtained. First, Phase D was mixed with Phase B. Subsequently, Phases A and C were mixed with one another and the mixture was filtered using a filter cushion (60S). The mixture of Phases D and B was then added into the mixture of Phases A and C while stirring and finally Phase E was added and the overall mixture was stirred at a constant speed.

### Example 2 Antiperspirant Soft Solid

| | |
|---|---|
| Cyclomethicone and Dimethicone Crosspolymer | 32 |
| Stearyl Dimethicone | 1 |
| Hydrogenated Castor Oil | 1 |
| Cyclomethicone | ad 100 |
| Watertight complex* | 0.5 |
| Dimethicone | 15 |
| Aluminum Zirconium Tetrachlorohydrex GLY | 24 |

| | |
|---|---|
| * 2/3 Acrylates/Octylacrylamide Copolymer 1/3 Hydrolyzed Jojoba Esters(and)Water | |

Weigh ingredients 1-5 into a vessel, heat to 80-85 °C and mix until waxes have melted and the mixture appears uniform. Remove from heat and add Dimethicone. The mixture will set at about 75 °C. Add Al-Zr-Tetrachlorohydrex after mixture cools below 75 °C. Continue mixing and cooling until the temperature is below 45 °C.

### Example 3 Body Mist

| **Phase A** | |
|---|---|
| Alcohol | ad 100 |
| Fragrance | 4.5 |

| **Phase B** | |
|---|---|
| Water | 16 |
| White Ginger Extract | 0.1 |

| **Phase C** | |
|---|---|
| Alcohol | 1.5 |
| Watertight complex* | 0.5 |

| **Phase D** | |
|---|---|
| Colorant | 0.2 |

| | |
|---|---|
| * see ex. 2 | |

Mix alcohol and fragrance with agitation. Add phase B with agitation and mix until clear and uniform. Filter the main batch and add separate mixed phase C to the main batch with agitation. Add phase D and mix until uniform. Example 4 **Clear Antiperspirant Gel (Female)**

| **Phase A** | |
|---|---|
| Cyclomethicone | 2.5 |
| Dimethicone | 0.5 |
| Cyclopentasiloxane(and)PEG/PPG-18/18 Dimethicone | 7.5 |
| Triethyl Citrate | 0.1 |
| Farnesol | 0.05 |

| **Phase B** | |
|---|---|
| Aluminum Zirconium Tetrachlorohydrex GLY | 54 |
| Water | ad 100 |
| Allantoin | 0.1 |
| Polysorbate 80 | 0.5 |
| Dipropylene Glycol | 21 |

| **Phase C** | |
|---|---|
| Fragrance | 0.5 |

| **Phase D** | |
|---|---|
| Watertight complex* | 0.5 |

| | |
|---|---|
| * see ex. 2 | |

Mix phase A and B ingredients separately. If the refractive index of phase A is higher than of phase B, add some water to phase B. Add phase B to phase A very slowly while rapidly mixing phase A and homogenize. Add phase C and mix until uniform. Add phase D while agitation.

### Example 4a

A gel is prepared as in example 4 whereby the watertight complex comprises 0.5 % Dermacryl^{®} AQF and 0.25 % Hydrolyzed Jojoba Esters.

### Example 5 Conditioner

| **Phase A** | |
|---|---|
| Water | ad 100 |
| Disodium EDTA | 0.02 |

| **Phase B** | |
|---|---|
| Steareth-20 | 1 |
| Dithicone Bisamino Hydroxypropyl Copolyol | 0.7 |
| Watertight complex* | 0.5 |
| Citric Acid | q.s. |

| **Phase C** | |
|---|---|
| Cetearyl Alcohol | 2 |
| Dipalmitoylethyldimonium Chloride | 3 |
| Glyceryl Stearate | 0.75 |

| **Phase D** | |
|---|---|
| Preservative | 0.5 |
| Fragrance | 1.5 |
| Sodium Hydroxide | q.s. |

| | |
|---|---|
| * see ex. 2 | |

Combine phase A ingredients with mixing and heat to 70 °C, maintain temperature. Add with mixing phase B ingredients one by one to phase A , mix until uniform and adjust pH by citric acid. Combine phase C ingredients and heat to 70 °C and mix to main batch until uniform. Add individually phase D ingredients and adjust pH with sodium hydroxide.

### Example 6 Lotion

| **Phase A** | |
|---|---|
| Water | ad 100 |
| Disodium EDTA | 0.05 |
| Glycerine | 2 |
| Carbomer^{®} | 0.3 |
| Pemulan^{®} | 0.1 |

| **Phase B** | |
|---|---|
| Tribehenin | 1.3 |
| DC 200/500 | 1 |
| Stearoxytrimethylsilane (and) Stearyl Alcohol | 0.7 |
| Diisostearyl Dimer Dilinoleate | 6 |
| BHT | 0.05 |
| VP/Eicosene Copolymer | 1 |
| Potassium Cetyl Phosphate | 0.5 |

| **Phase C** | |
|---|---|
| Vitamin A Acetate | 0.5 |

| **Phase D** | |
|---|---|
| Water | 2 |
| Triethanolamine | 0.4 |
| Watertight complex* | 1.5 |
| Cyclomethicone | 1 |
| D Panthenol L | 1 |

| **Phase E** | |
|---|---|
| Water | 2 |
| Phenonip | 0.5 |

| **Phase F** | |
|---|---|
| Fragrance | 0.8 |

| | |
|---|---|
| see ex. 2 | |

Mix water phase A ingredients separately and heat to 75-85 °C while mixing, maintain temperature. Mix phase B ingredients without Potassium Cetyl Phosphate (PCP) and heat to 75-80 °C, add PCP until free of lumps. Add melted phase C ingredients (45-50 °C) to phase B and add phase B (75 °C) to phase A. Homogenize for 7-10 minutes. Add from phase D water and TEA at 65 °C and after than the complex. At 50 °C add Cyclomethicone and than Panthenol. Add phase E and F at 35 °C and cool to 30 °C.

### Example 7 Shampoo

| **Phase A** | |
|---|---|
| Phylantriol&Disodium EDTA&Hydroxypropyl Guar&Hydroxypropyltrimonium Cloride& Tocopherylacetate (Miraspec^{®} KTS) | 55 |

| **Phase B** | |
|---|---|
| Fragrance | 1 |

| **Phase C** | |
|---|---|
| Plant extracts | 0.3 |
| Water | ad 100 |
| Watertight complex* | 1.8 |

| **Phase D** | |
|---|---|
| Colorant | 0.15 |

| **Phase E** | |
|---|---|
| Citric Acid (20 % solution) | q.s. |

| **Phase F** | |
|---|---|
| Ammonium Chloride (20 % solutione) | 2.4 |

| | |
|---|---|
| * both ingredients 1:1 | |

Mix separat prepared phases A and B. Add phase C ingredients while mixing. Add each ingredient of phase C separately to main main batch until clear and uniform. Continue mixing while adding phase D. Adjust pH with phase E if necessary. Add phase F while mixing.

### Example 8 Shower Gel

| **Phase A** | |
|---|---|
| Water | ad 100 |
| Disodium EDTA | 0.1 |
| Hydroxypropyl | 1 |
| DMDM Hydantoin | 0.3 |

| **Phase B** | |
|---|---|
| Sodium Laureth Sulfate | 10 |
| Disodium Laureth Sulfate | 20 |
| Cocamidopropyl Betaine | 9 |

| **Phase C** | |
|---|---|
| Preservative | 0.2 |
| Fragrance | 2 |
| Dye | 0.3 |

| **Phase D** | |
|---|---|
| Sodium Chloride (20 % solution) | 0.8 |

| **Phase E** | |
|---|---|
| Sodium Hydroxide (25 % solution) | q.s. |

| **Phase F** | |
|---|---|
| Dermacryl^{®} AQF | 0.5 |
| Hydrolyzed Jojoba Esters | 0.25 |

Mix separat prepared phases A and B. Add phase C ingredients while mixing. Add each ingredient of phase C separately to main main batch until clear and uniform. Adjust viscosity with phase D and continue mixing. Adjust pH with phase E while mixing. Add phase F while mixing.

### Beispiel 8a

A shower gel is prepared as in example 8 whereby the watertight complex comprises 0.5 % Dermacryl^{®} AQF and 0.25 % Hydrolyzed Jojoba Esters.

### Example 9 Eau de Toilette

| **Phase A** | |
|---|---|
| Alcohol | ad 100 |
| Fragrance | 17 |
| Benzophenone-2 | 0,1 |

| **Phase B** | |
|---|---|
| Water | 1,6 |

| **Phase C** | |
|---|---|
| Colorant | 0.3 |

| **Phase D** | |
|---|---|
| Dermacryl^{®} AQF | 5 |
| Hydrolyzed Jojoba Esters | 2 |

The proceeding is according to example 11.

### Example 10 Perfume

| | |
|---|---|
| Alcohol | ad 100 |
| Fragrance | 20 |
| Benzophenone 2 | 0.1 |
| Water | 9 |
| Watertight complex* | 5 |
| Colorant | 1 |

| | |
|---|---|
| * see ex. 2 | |

### Example 11 Eau de Toilette (EDT)

| **Phase A** | |
|---|---|
| Alcohol | ad 100 |
| Fragrance | 15 |
| Benzophenone 2 | 0.1 |

| **Phase B** | |
|---|---|
| Water | 3.6 |

| **Phase C** | |
|---|---|
| Alcohol | 10 |
| Watertight complex* | 3 |

| **Phase D** | |
|---|---|
| Colorant | 0.3 |

| | |
|---|---|
| * see ex. 2 | |

Mix separate prepared phases A and B. Mix alcohol and Hydrolyzed Jojoba Esters. Sprinkle in Acrylates/Octylacrylamide Copolymer while mixing and mix until clear and uniform. Add phase C to the main batch and after that phase D. Mix until uniform.

### Example 12 Clear Deodorant Stick

| **Phase A** | |
|---|---|
| Propylene Glycol | ad 100 |
| Sodium Stearate | 6.5 |
| Isosteareth-20 | 5 |
| Steareth-2 | 1 |

| **Phase B** | |
|---|---|
| 10% NaOH Sol | 0.1 |
| Water | 14.5 |
| Watertight complex* | 0.5 |

| **Phase C** | |
|---|---|
| Fragrance | 1.5 |
| Colorant | 0.4 |

| | |
|---|---|
| * see ex. 2 | |

### Example 13 Perfume

| **Phase A** | |
|---|---|
| Alcohol | 65 |
| Benzophenone-2 | 0.1 |
| Fragrance 13.5 | |

| **Phase B** | |
|---|---|
| Water | ad 100 |

| **Phase C** | |
|---|---|
| Colorant | 1,0 |

| **Phase D** | |
|---|---|
| Dermacryl^{®} AQF | 10 |
| Hydrolyzed Jojoba Esters | 5 |

Phases A and B are mixed and filtered. Phase C is added while mixing. Separately prepared phase D is added to the main batch and mixed until clear and uniform.

### Example 14 Perfume

| **Phase A** | |
|---|---|
| Alcohol | 55 |
| Benzophenone-2 | 0.1 |
| Fragrance | 20 |

| **Phase B** | |
|---|---|
| Water | ad 100 |

| **Phase C** | |
|---|---|
| Alcohol | 10 |
| Dermacryl^{®} AQF | 3,3 |
| Hydrolyzed Jojoba Esters | 1.7 |

| **Phase C** | |
|---|---|
| Colorant | 1,0 |

Phases A and B are mixed and filtered. Separately prepared phase C is added to the main batch and mixed until clear and uniform.
Phase C is added while mixing.

### Example 15 (Comparative test I)

Comparative tests were carried out using the composition according to Example 1 (Sample A) and the same composition without the ingredients of Phase D (Sample B).

Two drops (approx. 0.3g) of each sample were dropped on to the inner side of the right or left forearm of the test person. The drying time was 15 minutes. Two hours later, the forearm was immersed in water having a temperature of 26°C for 4 minutes. The forearm was then dried in the air. The immersion and drying process was repeated after 4 and 6 hours, rinsing the water container and changing the water in the container before each immersion.

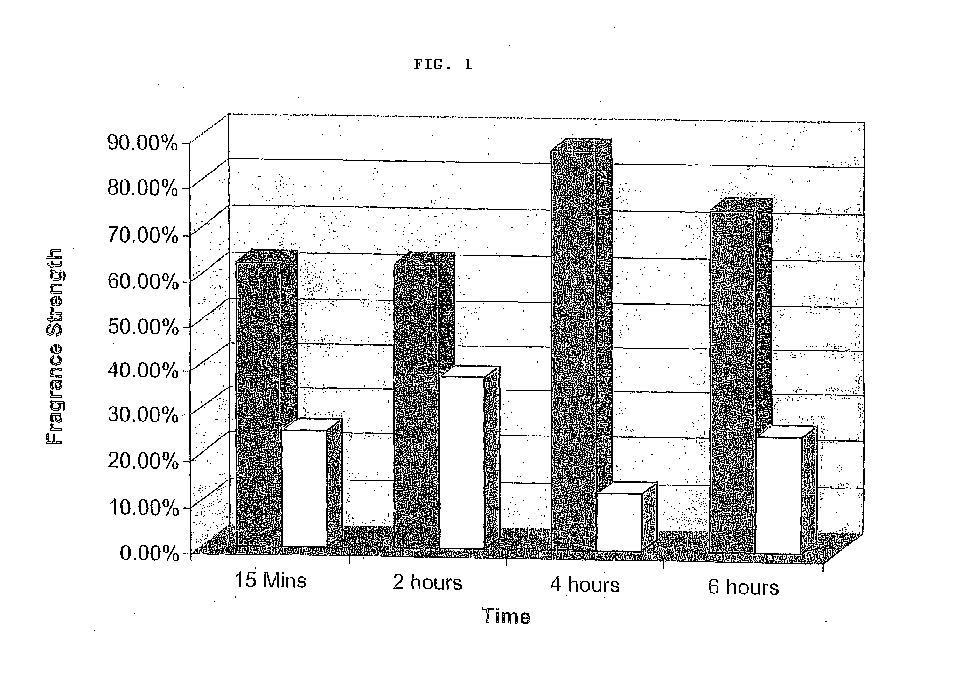

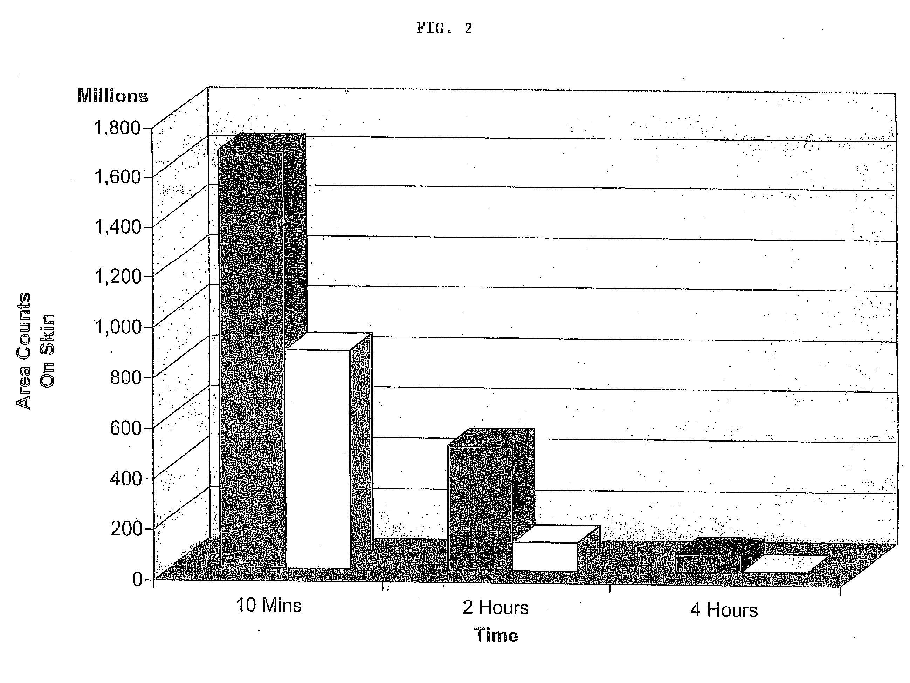

In a sensory test performed with 4 experienced testers, a result according to Fig. 1 was obtained. The same samples were analysed by means of gas chromatography/mass spectroscopy and the diagram according to Fig. 2 was obtained. The results were achieved by SPME (see example 16 part B). Both results demonstrate that the waterproofness is considerably increased.

In a comparative test using a EDT according to Example 11 (Sample C but with 0.5 % complex only) and the same EDT without the complex of copolymer and jojoba ester (Sample D), the waterproofness determined by means of a long-time sensory test was found to be more than twice the values of the same EDT without the complex according to the invention after 6 hours. A similar test using an aftershave demonstrated this superiority in a sensory test even after 8 hours.

### Example 16 (Comparative Test II)

### Part A

Comparative tests were carried out to demonstrate the ability of the complex of the invention to reduce the fragrance content in a perfume.

Two perfumes were produced. Perfume C had the following ingredients (in % by weight): 72.7 % Alcohol, 0.5 % Diisopropyl Adipate, 0.5 % C12-C15-Alkyl Lactate, 12 % Fragrance, 14.3 % Water. Perfume C1: the first three ingredients were the same as in Perfume C but with 9.6 % Fragrance, 15.3 % Water, 1 % complex of example 2, 0.4 % Na₂O₃ solution (10 %).

A group of 20 panelists were asked to identify the different fragrance shares in perfume C and perfume C1. Three panelists identified the different fragrances correctly, 17 did not.

The same results were obtained with a reduction of fragrance from 16 % to 12.8 % and from 15 % to 12 % respectively (each with 1 % of the complex in the formula with the reduced fragrance share).

### Part B SPME test

Solid-phase micro extraction (SPME) is a method in which analytes partition from a sample into a polymer, coated on a fused silica rod of typically 1 cm length by 100 µm diameter. The fiber is fastened into the end of a fine stainless steel tube contained in a syringe-like device and protected by an outer stainless steel needle. The device's plunger is depressed to expose the fiber to the sample matrix, retracted at the end of the sampling time, and then depressed again to expose the fiber to a desorption interface for analysis, in this case by Gas Chromatography. Reference is made to ex. 2 of US 2002/0192174 A1.

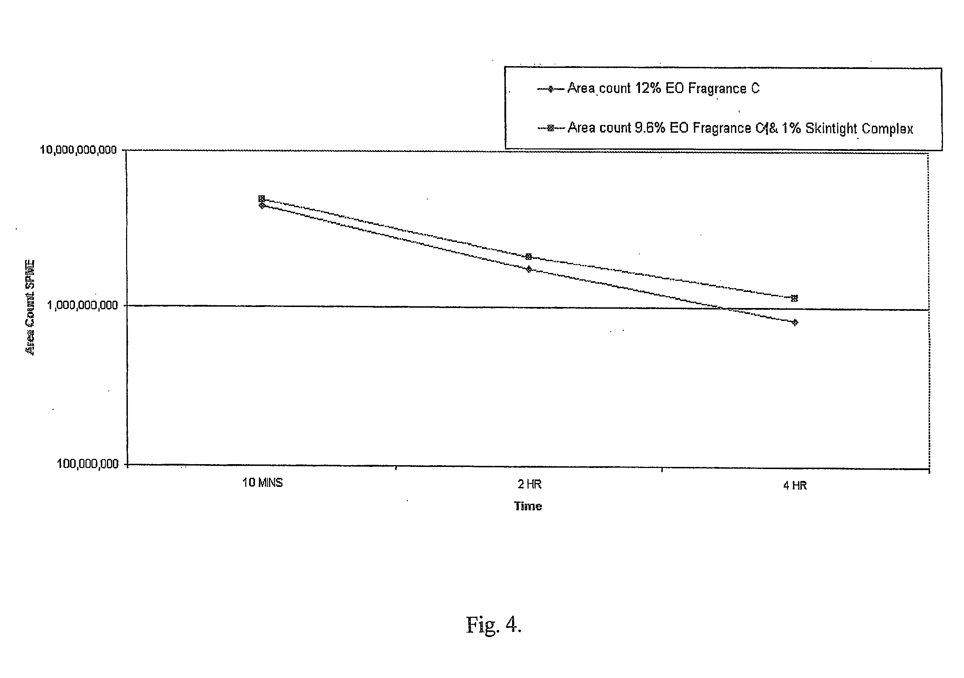

The result of the GC measurement is presented in Fig. 4 with 12 % perfume C and 9.6 % perfume C1 including 1 % of the watertight skintight complex according to example 2. Both perfumes show nearly the same area counts. This confirms the results of the consumer test of part A.

## Claims

1. A cosmetic composition with a watertight fragrance which composition comprises at least a fragrance and 0.1 to 15 % by weight of a fragrance-fixing complex which fragrance-fixing complex comprises 0.01 to 10% by weight of a hydrophobic, alcohol-soluble, carboxylated acrylates/octylacrylamide copolymer and
0.01 to 10% by weight of a hydrolyzed jojoba ester,
and where all percents are related to the total weight of the cosmetic composition.

2. The composition of claim 1 wherein additionally an alcohol or an alcohol/water mixture is present.

3. The composition of claim 1 wherein the amount of the copolymer is in the range of 0.1 to 5 % by weight.

4. The composition of claim 3 wherein the amount of the copolymer is in the range of 0.1 to 1 % by weight.

5. The composition of claim 1 wherein the amount of the jojoba ester is in the range of 0.1 to 5 % by weight.

6. The composition of claim 5 wherein the amount of the jojoba ester is in the range of 0.1 to 1 % by weight.

7. The composition of claim 1 wherein the fragrances are present in an amount ranging between 0.01 and 35 % by weight.

8. The composition of claim 7 wherein the fragrances are present in an amount ranging between 0.1 and 16 % by weight.

9. The composition of claim 8 wherein the fragrances are present in an amount ranging between 0.1 and 8 % by weight.

10. The composition of claim 1 wherein the hydrolyzed jojoba ester is one of plant origin (Simmondsia chinensis) having a saponification value according to AOCS Cd 3-25* of max. 1mg KOH/g, a iodine value according to AOCS Cd 1d-92* of between 14 and 17g/100g and a trans isomer value according to AOCS Cd 14-95* of max. 0.1% by weight wherein * means a test procedure according to American Oil Chemists Society.

11. The composition of claim 1 wherein the complex is provided in a cosmetic product selected from the group consisting of perfumes, Eau de Toilettes, hair products, body care products, sun care products, antiperspirants, deodorants, detergents and products of color cosmetic.

12. The composition of claim 1 wherein the share of the complex in the cosmetic product is in the range of 0.5 to 10 % by weight.

13. The composition of claim 1 wherein the share of the complex in the cosmetic product is in the range of 0.5 to 4 % by weight.

14. The composition of claim 1 wherein the share of the complex in the cosmetic product is in the range of 0.5 to 1.5 % by weight.

15. The composition of claim 1 wherein further cosmetic auxiliaries, cosmetic actives and carrier substances as they are commonly used in such preparations are ingredients of the composition.

## Patentansprüche

1. Kosmetische Zusammensetzung mit einer wasserbeständigen Parfümkomponente, **dadurch gekennzeichnet, dass** die Zusammensetzung wenigstens ein Parfümöl und 0,1 bis 15 Gew-% eines parfümfixierenden Komplexes umfasst, und der parfümfixierende Komplex
0,01 bis 1 Gew-% eines hydrophoben, alkohollöslichen, carboxylierten Acrylat/Octylacrylamid-Copolymeren und
0,01 bis 10 Gew-% eines hydrolysierten Jojobaesters umfasst,
wobei alle Prozente auf das Gesamtgewicht der kosmetischen Zusammensetzung bezogen sind.

2. Zusammensetzung nach Anspruch 1, **dadurch gekennzeichnet, dass** zusätzlich ein Alkohol oder Alkohol/Wasser-Gemisch vorhanden ist.

3. Zusammensetzung nach Anspruch 1, **dadurch gekennzeichnet, dass** der Anteil des Copolymeren im Bereich von 0,1 bis 5 Gew-% liegt.

4. Zusammensetzung nach Anspruch 3, **dadurch gekennzeichnet, dass** der Anteil des Copolymeren im Bereich von 0,1 bis 1 Gew-% liegt.

5. Zusammensetzung nach Anspruch 1, **dadurch gekennzeichnet, dass** der Anteil des Jojobaesters im Bereich von 0,1 bis 5 Gew-% liegt.

6. Zusammensetzung nach Anspruch 5, **dadurch gekennzeichnet, dass** der Anteil des Jojobaesters im Bereich von 0,1 bis 1 Gew-% liegt.

7. Zusammensetzung nach Anspruch 1, **dadurch gekennzeichnet, dass** der Anteil der Parfümöle im Bereich von 0,01 bis 35 Gew-% liegt.

8. Zusammensetzung nach Anspruch 7, **dadurch gekennzeichnet, dass** der Anteil der Parfümöle im Bereich von 0,1 bis 16 Gew-% liegt.

9. Zusammensetzung nach Anspruch 7, **dadurch gekennzeichnet, dass** der Anteil der Parfümöle im Bereich von 0,1 bis 8 Gew-% liegt.

10. Zusammensetzung nach Anspruch 1, **dadurch gekennzeichnet dass** der hydrolysierte Jojobaester aus pflanzlichem Ursprung ist (Simmondsia chinensis) mit einem Verseifungswert gemäß AOCS Cd 3-25* von maximal 1mg KOH/g, einem Iodwert gemäß AOCS Cd 1d-92* von 14 bis 17g/100g und einem Transisomerenwert gemäß AOCS Cd 14-95* von of maximal 0,1 Gew-%, wobei * ein Testverfahren der American Oil Chemists Society ist.

11. Zusammensetzung nach Anspruch 1, **dadurch gekennzeichnet, dass** der Komplex in einem kosmetischen Produkt vorliegt, ausgewählt aus der Gruppe, bestehend aus Parfüme, Eau de Toilettes, Haarprodukte, Körperpflegeprodukte, Sonnenschutzprodukte, Antischweißprodukte, Deodorantprodukte und Produkte der dekorativen (Farb-)Kosmetik.

12. Zusammensetzung nach Anspruch 1, **dadurch gekennzeichnet, dass** der Anteil des Komplexes in dem kosmetischen Produkt im Bereich von 0,5 bis 10 Gew-% liegt.

13. Zusammensetzung nach Anspruch 1, worin der Anteil des Komplexes in dem kosmetischen Produkt im Bereich von 0,5 bis 4 Gew-% liegt.

14. Zusammensetzung nach Anspruch 1, worin der Anteil des Komplexes in dem kosmetischen Produkt im Bereich von 0,5 bis 1,5 Gew-% liegt.

15. Zusammensetzung nach Anspruch 1, **dadurch gekennzeichnet, dass** Bestandteile der Zusammensetzung weitere kosmetische Hilfsstoffe, Wirkstoffe und Trägerstoffe enthalten sind, wie sie üblicherweise in solchen Zusammensetzungen verwendet werden.

## Revendications

1. Composition cosmétique avec une fragrance résistant à l'eau, laquelle composition comprend au moins une fragrance et 0,1 à 15% en poids d'un complexe fixant la fragrance, lequel complexe fixant la fragrance comprend 0,01 à 10% en poids d'un copolymère hydrophobe, soluble dans un alcool, acrylates carboxylés/octylacrylamide, et
0,01 à 10% en poids d'un ester de jojoba hydrolysé,
et où tous les pourcentages sont par rapport au poids total de la composition cosmétique.

2. Composition selon la revendication 1, dans laquelle est présent en outre, un alcool ou un mélange alcool/eau.

3. Composition selon la revendication 1, dans laquelle la quantité du copolymère se situe dans l'intervalle allant de 0,1 à 5% en poids.

4. Composition selon la revendication 3, dans laquelle la quantité du copolymère se situe dans l'intervalle allant de 0,1 à 1% en poids.

5. Composition selon la revendication 1, dans laquelle la quantité de l'ester de jojoba se situe dans l'intervalle allant de 0,1 à 5% en poids.

6. Composition selon la revendication 5, dans laquelle la quantité de l'ester de jojoba se situe dans l'intervalle allant de 0,1 à 1% en poids.

7. Composition selon la revendication 1, dans laquelle les fragrances sont présentes en une quantité se situant dans l'intervalle allant de 0,01 à 35% en poids.

8. Composition selon la revendication 7, dans laquelle les fragrances sont présentes en une quantité se situant dans l'intervalle allant de 0,1 à 16% en poids.

9. Composition selon la revendication 8, dans laquelle les fragrances sont présentes en une quantité se situant dans l'intervalle allant de 0,1 à 8% en poids.

10. Composition selon la revendication 1, dans laquelle l'ester de jojoba hydrolysé est d'origine végétale (*Simmondsia chinensis*) ayant une valeur de saponification selon AOCS Cd 3-25* de maximum 1 mg KOH/g, une valeur d'iode selon AOCS Cd 1d-92* allant de 14 à 17 g/100 g et une valeur d'isomère trans selon AOCS Cd 14-95* de maximum 0,1% en poids, où * signifie une procédure de test selon l'American Oil Chemists Society.

11. Composition selon la revendication 1, dans laquelle le complexe est proposé dans un produit cosmétique choisi parmi le groupe consistant en les parfums, les eaux de toilette, les produits capillaires, les produits pour le soin du corps, les produits de soin solaire, les antiperspirants, les déodorants, les détergents et les produits cosmétiques colorés.

12. Composition selon la revendication 1, dans laquelle la part du complexe dans le produit cosmétique se situe dans la gamme allant de 0,5 à 10% en poids.

13. Composition selon la revendication 1, dans laquelle la part du complexe dans le produit cosmétique se situe dans la gamme allant de 0,5 à 4% en poids.

14. Composition selon la revendication 1, dans laquelle la part du complexe dans le produit cosmétique se situe dans la gamme allant de 0,5 à 1,5% en poids.

15. Composition selon la revendication 1, dans laquelle de plus, des auxiliaires cosmétiques, des agents actifs cosmétiques et des substances support, tels qu'ils sont habituellement utilisés dans de telles préparations, sont des ingrédients de la composition.
